# FASCICULE DE BREVET EUROPEEN

(11) **EP 1 536 697 B1**
(45) Date de publication et mention de la délivrance du brevet: **21.02.2007**
(21) Numéro de dépôt: 03769612.7
(22) Date de dépôt: 11.09.2003
(51) Int. Cl.: A23L 1/30, A61K 36/30

(54) **COMPOSITION COMPRENANT EN ASSOCIATION AU MOINS UNE HUILE DE COURGE ET AU MOINS UNE HUILE DE BOURRACHE, SON UTILISATION COMME MEDICAMENT, COMME AGENT DERMATOLOGIQUE OU DERMATO-COSMETIQUE**
ZUSAMMENSETZUNGEN ENTHALTEND MINDESTENS KÜRBISÖL UND BORRETSCHÖL, DEREN VERWENDUNG ALS ARZNEIMITTEL, DERMATIKUM ODER DERMATOKOSMETISCHE ZUSAMMENSETZUNG
COMPOSITION CONTAINING IN COMBINATION AT LEAST ONE GOURD OIL AND AT LEAST ONE BORAGE OIL, USE THEREOF AS MEDICINE, AS DERMATOLOGICAL OR DERMATO-COSMETIC AGENT

(30) Priorité: 12.09.2002 FR 0211351
(43) Date de publication de la demande: 08.06.2005
(73) Titulaire: Schwaller, Marc, 94210 La Varenne (FR)
(72) Inventeur: Schwaller, Marc, 94210 La Varenne (FR)
(74) Mandataire: Ahner, Francis
(86) Numéro de dépôt international: PCT/FR2003/002693
(87) Numéro de publication internationale: WO 2004/023897

(56) Documents cités:
- WO-A-00/44863
- WO-A-01/37849
- BE-A- 1 011 858
- FR-A- 2 613 933
- FR-A- 2 663 848

## Description

La présente invention concerne une nouvelle composition comprenant en association au moins une huile de courge, au moins une huile de bourrache, et éventuellement au moins un composé choisi parmi un extrait de soja et un extrait d'ortie. L'invention a également pour objet une telle composition pour son utilisation comme médicament, comme agent dermatologique ou dermo-cosmétique, ou comme nutraceutique (complément alimentaire), notamment pour son utilisation dans la prévention ou le traitement de l'alopécie, de l'acné, de l'hirsutisme, de la séborrhée ou des odeurs corporelles.

La courge, du nom latin *Cucurbita pepo*, appartient à la famille des Cucurbitacées. La courge est une grande plante herbacée annuelle à tige anguleuse, très rameuse. Les feuilles sont simples, alternes, longuement pétiolées. Les fleurs, jaunes, sont unisexuées. Le fruit est une grosse baie volumineuse ou oblongue à pulpe charnue, spongieuse, et est de couleur variable. Les graines sont nombreuses, plates et ovoïdes. Les pépins sont composés chimiquement en moyenne de 35 à 50% d'huile, de 25 à 40% de protéines, de 10% de pectines et de 4 à 5% d'éléments minéraux. L'huile, qui est riche en tocophérols, caroténoïdes, en acides gras insaturés (70 à 80% des acides gras totaux) et en phytostérols, est la partie la plus intéressante, et est couramment utilisée, notamment pour son intérêt nutritionnel.

A côté de cet usage alimentaire, l'huile de courge est classiquement utilisée *per os* en complément de traitement des troubles fonctionnels prostatiques et des difficultés de diurèse. On dit ainsi que l'huile de courge agit comme décongestionnant prostatique. Parmi les troubles fonctionnels prostatiques, on peut notamment citer l'hypertrophie bénigne de la prostate ou l'adénome prostatique. L'huile de courge est par ailleurs utilisée comme anti-parasitaire ou comme anthelminthique.

BE 1 011 858 décrit des produits de soin du corps externes cosmétiques et dermatologiques, comprenant en association des huiles végétales liposolubles et des extraits de plantes liposolubles.

La demanderesse a découvert de manière surprenante que l'association d'une huile extraite de courge à une huile extraite de bourrache, dans des compositions telles que des compositions pharmaceutiques, dermatologiques, cosmétiques, ou alimentaires, permettait de traiter divers troubles et affections cutanés, et ceci de manière beaucoup plus efficace que les compositions utilisées jusqu'à présent, en particulier les compositions contenant de l'huile de courge comme seul actif

La bourrache, du nom latin *Borago officinalis,* appartient à la famille des boraginacées. La bourrache est une plante herbacée annuelle à grandes feuilles basales et à belles fleurs bleues. L'huile des graines de bourrache est riche en acides gras polyinsaturés, notamment en acide gamma-linolénique, et a déjà été utilisée pour améliorer l'hydratation de la peau, soigner les douleurs prémenstruelles, les rhumatismes ou encore l'eczéma. L'huile de bourrache n'a pourtant jamais été utilisée en association avec l'huile de courge dans des compositions pour la peau ou le cuir chevelu.

Or, la demanderesse a montré que les actifs suivants : huile de courge et huile de bourrache, auxquels peut être ajouté au moins un autre composé choisi parmi un extrait d'ortie, un extrait de soja, ou leurs mélanges, pouvaient être utilisés en association dans des compositions telles que des compositions pharmaceutiques, dermatologiques, cosmétiques, nutraceutiques ou alimentaires, notamment dans la prévention ou le traitement de l'alopécie, de l'acné, de l'hirsutisme, de la séborrhée ou des odeurs corporelles.

Les huiles de courge et de bourrache selon la présente invention, ainsi que les extraits d'ortie et de soja, sont des produits naturels, et s'avèrent être des composés, cosmétiquement, pharmaceutiquement, dermatologiquement et nutraceutiquement acceptables, non agressifs, ni toxiques, ni irritants pour la peau, hypoallergéniques, apaisants, hydratants et anti-inflammatoires pour la peau. De plus, ces composés sont obtenus par des procédés d'extraction classique, et sont disponibles commercialement.

La présente invention a ainsi pour objet une composition comprenant en association :
(a) au moins une huile de courge, et
(b) au moins une huile de bourrache.

Avantageusement selon la présente invention, la composition comprend en outre au moins un composé choisi parmi un extrait d'ortie (c) et un extrait de soja (d). La composition selon la présente invention peut ainsi contenir en tant qu'agents actifs une huile de courge (a) et une huile de bourrache (b), ou encore une huile de courge (a) et une huile de bourrache (b) en association avec soit un extrait d'ortie (c), soit un extrait de soja (d), soit un extrait d'ortie (c) et un extrait de soja (d).

Dans un mode de réalisation particulier de la présente invention, l'huile de courge (a) est une huile extraite de pépins de courge et/ou l'huile de bourrache (b) est une huile extraite de graines de bourrache. Les procédés d'extraction de ces deux types d'huile sont classiques.

L'huile de pépins de courge et l'huile de bourrache peuvent ainsi être obtenues par différents procédés, utilisés séparément ou en combinaison :
- par simple pression des pépins ou graines à froid, permettant de recueillir tous les composants lipophiles présents dans les pépins ;
- par des solvants organiques tels que l'éthanol, le méthanol, le dichlorométhane, le chloroforme, et l'hexane, pouvant entraîner une certaine sélectivité dans l'extraction ;
- par le gaz carbonique à l'état supercritique.

La composition selon l'invention peut mettre en oeuvre une huile obtenue par l'un ou l'autre de ces procédés, avec une préférence pour la pression à froid qui est la seule méthode garantissant d'obtenir tous les composants lipophiles de la graine, et garantissant également l'absence d'une éventuelle dégradation de certains composants par le chauffage nécessaire à l'évaporation des solvants.

Dans un autre mode de réalisation particulier de la présente invention, l'extrait d'ortie (c) est un extrait de racines d'ortie, avantageusement sous forme de poudre. Cet extrait est classiquement obtenu par macération d'orties dans un solvant pouvant être l'eau, un solvant type éthanol, hexane, ou avantageusement un mélange hydro-alcoolique, puis transformation en poudre par atomisation.

Dans un autre mode de réalisation particulier de la présente invention, l'extrait de soja (d) est de la lécithine de soja, avantageusement obtenue à partir d'huile de graines de soja, ou un extrait riche en isoflavones, avantageusement sous forme de poudre. L'extrait riche en isoflavones est avantageusement un extrait hydro-alcoolique de soja. Par le terme d"'extrait riche en isoflavones", on entend au sens de la présente invention, un extrait contenant au moins 2% en poids, de préférence de 10 à 40% en poids, d'isoflavones par rapport au poids total de l'extrait.

Selon une caractéristique particulière de la présente invention, l'huile de courge (a) est présente à une concentration comprise entre 20 et 40 %, avantageusement entre 25 et 35 % en poids, par rapport au poids total de la composition.

Selon une autre caractéristique particulière de la présente invention, l'huile de bourrache (b) est présente à une concentration comprise entre 10 et 30 %, avantageusement entre 15 et 30 % en poids, par rapport au poids total de la composition.

Selon une autre caractéristique particulière de la présente invention, l'extrait d'ortie (c) est présent à une concentration comprise entre 0 et 60 %, avantageusement entre 25 et 60%, de manière encore plus avantageuse entre 35 et 55 % en poids, par rapport au poids total de la composition.

Selon une autre caractéristique particulière de la présente invention, l'extrait de soja (d) est présent à une concentration comprise entre 0 et 10 %, avantageusement entre 0 et 5 % en poids, par rapport au poids total de la composition.

Dans un mode de réalisation particulier de la présente invention, la composition est formulée pour être administrée par voie orale. La composition peut ainsi se présenter sous forme de capsule, gélule, comprimé, granule, pâte à mâcher, gomme, huile ou gelée huileuse. Lorsque la composition se présente sous forme de capsule molle ou de gélule, l'enveloppe de ces capsules molles ou de ces gélules peut être constituée de gélatine animale ou d'un matériau d'origine végétale (dérivé de cellulose ou d'amidon, protéine végétale). Lorsque la composition se présente sous forme de gélule, de comprimé, ou de granule, le mélange d'actifs peut être fixé sur un support pulvérulent tel que la silice, la cellulose, et la maltodextrine.

Avantageusement selon la présente invention, la composition est une composition orale, contenant le mélange : huile de pépins de courge (a), huile de bourrache (b), extrait de racine d'ortie (c), et lécithine de soja (d) et, si nécessaire, des additifs technologiques tels que des épaississants ou des anti-oxydants, et se présente avantageusement sous forme de capsule molle ou de gélule.

La composition peut également être une composition ou un complément alimentaire, telle qu'une barre de céréales, une poudre à diluer dans l'eau du type café, thé ou chocolat instantané, une crème dessert, et de la margarine. Dans ce cas, le mélange d'actifs peut être fixé sur un support pulvérulent.

Dans un autre mode de réalisation particulier de la présente invention, la composition est formulée pour être administrée par voie topique ou rectale. Dans ce cas, et lorsque l'extrait d'ortie (c) est sous forme de poudre, la fraction de poudre d'ortie non dissoute dans le mélange d'huile est avantageusement éliminée. La composition selon la présente invention peut ainsi être appliquée sur la peau ou sur le cuir chevelu, et se présenter sous forme de crème, de pommade, ou d'huile. La composition peut également être une composition rectale, se présentant notamment sous forme de suppositoires, ou de tubes canules.

De manière avantageuse selon la présente invention, lorsque l'extrait d'ortie est sous forme de poudre, la composition est susceptible d'être obtenue selon le procédé comprenant l'étape de macération de la poudre d'ortie (c) dans un mélange d'huile de courge (a) et d'huile de bourrache (b), éventuellement additionné de lécithine de soja. Quel que soit le mélange utilisé pour la macération (mélange d'huiles ou mélange d'huiles et de lécithine de soja), si un extrait de soja riche en isoflavones est utilisé, il sera ajouté après macération.

Avantageusement selon la présente invention, la composition est titrée de manière à permettre l'administration d'une dose journalière de 10 mg à 5 g, de préférence d'environ 400 mg, d'huile de courge (a) par jour, de 10 mg à 5 g, de préférence d'environ 300 mg, d'huile de bourrache (b) par jour, de 10 mg à 5 g, de préférence d'environ 400 mg, d'extrait d'ortie (c) par jour, et de 5 mg à 10 g d'extrait de soja (d) par jour, de préférence d'environ 40 mg lorsque l'extrait de soja (d) est de la lécithine de soja.

Avantageusement selon la présente invention, la composition est une composition pharmaceutique, dermatologique, cosmétique, nutraceutique ou alimentaire, et peut comprendre tout véhicule ou excipient approprié, acceptable du point de vue pharmaceutique, dermatologique, cosmétique, ou nutraceutique, ainsi que des additifs conventionnels, connus de l'homme du métier.

La composition selon la présente invention peut contenir d'autres agents actifs tels qu'une huile de Serenoa repens ayant une activité inhibitrice de la 5-alpha réductase ; un extrait de thé vert riche en catéchols, anti-oxydant et permettant de lutter contre l'apoptose des follicules pileux ; un sel de zinc, tel que l'acétate, le chlorure, le citrate, le gluconate, le lactate, l'oxyde, le carbonate ou le sulfate de zinc, ou une forme chélatée du zinc telle que le chélate zinc-acide aminé, ayant également une activité inhibitrice de la 5-alpha réductase ; des vitamines du groupe B, telles que la vitamine B1 ou thiamine (notamment sous forme de chlorhydrate ou de mononitrate), la vitamine B2 ou riboflavine (notamment sous forme pure ou sous forme de phosphate de sodium), la vitamine B3 ou niacine (notamment sous forme d'acide nicotinique ou de nicotinamide), la vitamine B5 ou acide pantothénique (notamment sous forme de pantothénate de calcium ou de sodium ou sous forme de dexpantothénol), la vitamine B6 ou pyridoxine (notamment sous forme de chlorhydrate ou de phosphate), la vitamine B12 ou cobalamine (notamment sous forme de cyanocobalamine et d'hydroxocobalamine), la vitamine H ou biotine ; et les acides aminés soufrés, pouvant jouer un rôle important de nutriment pour le bulbe pileux (méthionine et cystine).

La présente invention a également pour objet les compositions décrites ci-dessus pour leur utilisation comme médicaments, comme agent dermatologiques ou dermo-cosmétiques, ou comme nutraceutiques (compléments alimentaires).

La présente invention a également pour objet les compositions décrites ci-dessus pour leur utilisation dans la prévention ou le traitement de l'alopécie, de l'acné, de l'hirsutisme, de la séborrhée ou des odeurs corporelles.

La chute des cheveux peut être provoquée par des causes diverses et peut engendrer un simple désagrément d'ordre esthétique ou constituer une véritable pathologie. La composition selon la présente invention permet de traiter différentes formes d'alopécie existantes.

Avant d'évoquer les différentes formes d'alopécie existantes, rappelons que le bulbe capillaire évolue en permanence entre 3 phases : anagène (phase de croissance), télogène (phase de repos) et catagène (phase de dégénérescence). A l'état normal, plus de 90% des cheveux sont au stade anagène. La « perte de cheveux » est la résultante de deux mécanismes physiologiques souvent corrélés : d'une part l'augmentation anormale du pourcentage (20 à 40% au lieu de 10%) de cheveux en phase télogène, les « cheveux » télogènes se limitant alors à un « duvet », et d'autre part la dégénérescence du bulbe (phase catagène) conduisant à la mort et à la chute du cheveu.

Ces mécanismes physiologiques semblent avoir plusieurs origines :
- hormonale : le bulbe capilaire est très sensible aux androgènes et notamment aux dérivés de la testostérone. Un excès d'activité de la 5-alpha réductase, enzyme transformant la testostérone en dihydroTestostérone, forme plus active au plan hormonal, semble favoriser le passage des cheveux en phase télogène. L'alopécie androgénétique a pour origine une activité enzymatique anormalement élevée de la 5-alpha réductase au niveau du cuir chevelu. C'est sur la base de cette constatation qu'à été développé le médicament le plus récent contre l'alopécie : le Finastéride, qui est un inhibiteur de la 5-alpha réductase.
- circulatoire : une diminution de la microcirculation sanguine au niveau du bulbe capillaire entraîne le ralentissement de sa croissance voire sa dégénérescence. C'est le mécanisme d'action reconnu d'un médicament largement utilisé dans l'alopécie : le Minoxidil, qui est un vasodilatateur périphérique.
- immunologique : une chute rapide et importante des cheveux peut être provoquée par une réaction auto-immune contre le bulbe capillaire : il s'agit de l'alopécie diffuse ou pelade (alopecia aerata) qui peut rester localisée ou se généraliser à tout le cuir chevelu (alopecia totalis), voire à tous les poils du corps (alopecia universalis). Son traitement médicamenteux actuel consiste essentiellement en des anti-inflammatoires (corticoïdes) et en des immuno-suppresseurs (ciclosporine).

La présente invention étant une association de plusieurs substances naturelles d'origine végétale, il est probable que son efficacité clinique soit la résultante de plusieurs mécanismes d'action, dont les principaux sont :
- régulation de l'excès de 5-alpha réductase: il est en effet démontré que plusieurs composants de la formule ont une activité inhibitrice de la 5-alpha réductase (l'huile de courge, la racine d'ortie et l'huile de bourrache). Cette activité est liée d'une part aux phytostérols que contiennent ces 3 extraits végétaux, mais aussi aux acides gras insaturés contenus dans les deux huiles ; cette action pourrait résulter d'une modification de l'environnement membranaire de l'enzyme.
- activité anti-inflammatoire : principalement par les acides gras insaturés des deux huiles.

Ce double mécanisme d'action permet d'expliquer l'activité observée dans le traitement de différentes formes d'alopécie : alopécie androgénétique et alopécie diffuse (pelade), mais également dans d'autres pathologies de la peau ayant à la fois une origine hormonale ou inflammatoire : acné, hirsutisme, séborrhée et odeurs corporelles.

Les exemples suivants sont destinés à illustrer l'invention sans aucunement en limiter la portée. A moins qu'il n'en soit précisé autrement, les pourcentages indiqués dans les exemples suivants sont des pourcentages en poids.

### Exemples de compositions selon la présente invention et de leurs procédés de préparation:

### Exemple 1 : Composition, se présentant sous forme de capsule, contenant un macérat de poudre de racines d'ortie (c) dans le mélange d'huile de pépins de courge (a), d'huile de graines de bourrache (b), et de lécithine de soja (d) - procédé de préparation

### a) Solubilisation des composants lipophiles de la poudre de racines d'ortie dans le mélange d'huile de pépins de courge, d'huile de bourrache et de lécithine :

| | |
|---|---|
| Huile de pépins de courge : | 100 kg |
| Poudre de racines d'ortie : | 200 kg |
| Huile de bourrache : | 75 kg |
| Lécithine : | 10 kg |

Dans une cuve chauffante dans laquelle est réalisé un vide partiel :
- mélanger l'huile de pépins de courge, l'huile de bourrache, la lécithine et éventuellement un anti-oxydant (acétate de tocophérol par exemple), puis porter la température à 70°C,
- introduire sous agitation la poudre de racine d'ortie broyée finement (granulométrie inférieure à 300µm), et maintenir sous agitation pendant 2 heures,
- refroidir à température ambiante sous agitation, puis éventuellement
- conditionner le mélange obtenu en fût étanche sous atmosphère d'azote.

### b) Fabrication des capsules molles

Le procédé de fabrication des capsules molles est tout à fait standardisé : le mélange décrit ci-dessus peut être injecté directement au moment du scellage à chaud des deux coques en gélatine de la capsule molle dans des moules rotatifs appropriés. L'enveloppe scellée des capsules molles est ensuite séchée dans un courant d'air chaud, une faible quantité de lubrifiant étant généralement ajoutée pour éviter l'adhésion des capsules molles entre elles.
A titre d'exemple, chaque capsule molle peut contenir :

| | |
|---|---|
| Huile de pépins de courge : | 200 mg |
| Poudre de racines d'ortie : | 400 mg |
| Huile de bourrache : | 150 mg |
| Lécithine : | 20 mg |

### Exemple 2 : Composition, se présentant sous forme de capsule, contenant un mélange d'huile de pépins de courge (a), d'huile de graines de bourrache (b), d'extrait de racines d'ortie (c), et de lécithine de soja (d) - procédé de préparation de la composition par simple mélange des composants

### a) Mélange des composants :

| | |
|---|---|
| Huile de pépins de courge : | 100 kg |
| Extrait de racines d'ortie : | 100 kg |
| Huile de bourrache : | 75 kg |
| Lécithine : | 10 kg |

Dans une cuve munie d'un système d'agitation :
- introduire successivement l'huile de pépins de courge, l'huile de bourrache et la lécithine ; un chauffage modéré (40 à 50 ° C) et/ou un préchauffage préalable de la lécithine peuvent être nécessaires pour obtenir une parfaite homogénéisation de cette dernière,
- en maintenant l'agitation, incorporer progressivement l'extrait de racine d'ortie, puis éventuellement
- conditionner le mélange obtenu en fût étanche sous atmosphère d'azote.

### b) Fabrication des capsules molles

Le procédé de fabrication des capsules molles est identique à celui de l'exemple 1, la composition par capsule étant de :

| | |
|---|---|
| Huile de pépins de courge : | 200 mg |
| Extrait de racines d'ortie : | 200 mg |
| Huile de bourrache : | 150 mg |
| Lécithine : | 20 mg |

### Evaluation de l'activité induite par l'administration de compositions selon la présente invention pour lutter contre l'alopécie chez l'homme

### 1. Protocole expérimental

Une étude clinique a été réalisée sur 70 volontaires de sexe masculin, présentant une alopécie de type androgénétique, qui suivirent pendant 6 mois un traitement consistant à la prise matin et soir d'une capsule contenant l'une des compositions ci-dessous :
- Groupe 1 : Huile de pépins de courge : 200 mg.
- Groupe 2 : Extrait de racines d'ortie : 200 mg.
- Groupe 3 : Huile de bourrache: 150 mg.
- Groupe 4 : Lécithine de soja : 20 mg.
- Groupe 5 :
   * Huile de Pépins de Courge : 200 mg, et
   * Huile de bourrache : 150 mg.
- Groupe 6 :
   * Huile de Pépins de Courge : 200 mg,
   * Extrait de Racine d'Ortie: 200 mg, et
   * Huile de bourrache : 150 mg.
- Groupe 7 :
   * Huile de Pépins de Courge : 200 mg,
   * Extrait de Racines d'Ortie : 200 mg,
   * Huile de bourrache: 150 mg, et
   * Lécithine de soja : 20 mg.
   Simple mélange des 4 composants.
- Groupe 8 :
   * Huile de Pépins de Courge: 200 mg,
   * Huile de Bourrache : 150 mg,
   * Lécithine de Soja : 20 mg, et
   * Poudre de Racines d'Ortie : 400 mg.
   Macération à 70°C pendant 2h de la poudre de Racines d'Ortie dans le mélange : Huile de Pépins de Courge + Huile de bourrache + Lécithine.

### 2. Tests réalisés

Pour chaque volontaire, un trichogramme a été réalisé sur la même zone de 1 cm² de cuir chevelu avant la participation à l'étude, et après 3 mois de traitement : il a permis de déterminer le nombre total de cheveux et le nombre de cheveux en phase anagène (cheveux anagènes/cm²).

A partir de ces valeurs expérimentales, il était possible de calculer le nombre de cheveux en phase télogène (cheveux télogènes/cm² = nombre de cheveux/cm² - cheveux anagènes/cm²) et d'en déduire le ratio anagènes / télogènes (nombre de cheveux en phase anagène/nombre de cheveux en phase télogène).

### 3. Résultats

Les résultats du nombre de cheveux en phase télogène et du ratio anagènes / télogènes sont exposés dans le tableau 1 suivant :

**Tableau 1 :**

| | **Nombre total de cheveux/cm²** | | | **Ratio anagènes / télogènes** | | |
|---|---|---|---|---|---|---|
| | Avant traitement | Après traitement | Evolution | Avant traitement | Après traitement | Evolution |
| 1 | 167,2 | 171,2 | 2,4% | 1,96 | 2,16 | +10,2% |
| | +/-3,0 | +/-4,9 | NS | +/-0,14 | +/-0,22 | NS |
| 2 | 162,8 | 166,6 | +2,3% | 2,02 | 2,30 | +13,9% |
| | +/- 7,2 | +/- 9,6 | NS | +/-0,21 | +/-0,28 | NS |
| 3 | 168,4 | 171,6 | + 1,9% | 2,18 | 2,26 | +3,7% |
| | +/- 4,3 | +/- 4,5 | NS | +/-0,17 | +/-0,22 | NS |
| 4 | 167,0 | 168,6 | +0,9% | 2,02 | 2,12 | +4,9% |
| | +/-5,4 | +/-3,7 | NS | +/-0.15 | +/-0,14 | NS |
| 5 | 166,6 | 172,2 | +3,4% | 2,04 | 2,46 | +20,5% |
| | +/-9,4 | +/-8,7 | S (p<0,05) | +/-0,41 | +/-0,34 | S (p<0,05) |
| 6 | 164,8 | 169,8 | +3,0% | 2,16 | 2,60 | +20,3% |
| | +/-6,8 | +/-9,1 | S (p<0,05) | +/-0,22 | +/-0,14 | S (p<0,05) |
| 7 | 163,2 | 169,6 | + 3,9% | 1,92 | 2,36 | +22,9% |
| | +/- 9,3 | +/- 8,3 | S (p <0,05) | +/-0,18 | +/-0,28 | S (p<0,01) |
| 8 | 167,0 | 175,0 | + 4,8% | 1,90 | 2,48 | +30,5% |
| | +/- 7,5 | +/- 3,2 | S (p <0,05) | +/-0,20 | +/-0,20 | S (p<0,01) |

On observe que le nombre de cheveux est très légèrement augmenté après traitement avec l'huile de pépins de courge, l'huile de bourrache et l'extrait de Racine d'Ortie seuls (groupes 1, 2 et 3). Cette augmentation devient statistiquement significative avec le groupe 5 associant l'huile de pépins de courge et l'huile de bourrache.

Le ratio anagènes/télogènes augmente après traitement avec l'huile de pépins de courge, l'huile de bourrache et l'extrait de Racine d'Ortie seuls (groupes 1, 2 et 3), mais de manière non significative. L'augmentation est substantielle et significative pour les groupes 5 à 8 après traitement.

Il est intéressant de noter que l'augmentation la plus importante de ce ratio est obtenue pour le groupe 8, mettant en oeuvre une macération de la poudre de Racines d'Ortie dans le mélange huile de Pépins de Courge, huile de bourrache et Lécithine de Soja.

Enfin, il a été observé à l'issue du traitement qu'une rémanence de l'effet se manifestait, une reprise de la chute des cheveux n'apparaissant, en moyenne, que 1 à 3 mois après l'arrêt du traitement. Cet aspect très intéressant différencie la présente invention de la plupart des traitements disponibles à ce jour.

### 4. Conclusions

Les tests cliniques (nombre total de cheveux/cm² et ratio anagène / télogène) démontrent ainsi une activité très modérée de l'huile de pépins de courge seule (400mg/j), de l'extrait de racines d'ortie seul (400mg/j), de l'huile de bourrache (300mg/j) seule ou encore de la lécithine de soja seule (40mg/j).

Par contre, de façon tout à fait inattendue, en associant l'huile de courge avec l'huile de bourrache, auxquelles peuvent être rajoutés un extrait de racines d'ortie et/ou un extrait de soja, une activité anti-alopécie réelle a pu être observée.

De façon encore plus surprenante, en procédant à une macération d'une poudre de racine d'ortie dans le mélange : huile de pépins de courge - huile de bourrache - lécithine, le produit obtenu développait une activité clinique encore plus importante à la même posologie. Après avoir vérifié l'absence d'activité de la lécithine à la dose testée, force a été de constater que ce procédé original de fabrication, comprenant l'étape de macération de poudre d'ortie dans le mélange d'huile ou dans le mélange d'huiles et de lécithine, conférait à cette préparation des propriétés inattendues.

Le fait que l'activité anti-alopécie se manifeste de manière plus importante lorsque les composants sont mis en oeuvre selon le procédé ci-dessus peut avoir plusieurs explications et notamment pourrait être lié :
- à une incapacité de l'organisme humain à assimiler certains composants hydrophobes présents dans l'huile de pépins de courge, ou dans la poudre de racines d'ortie administrée seule, alors que le pouvoir solubilisant et complexant (formation de micelles) de la lécithine permettrait une meilleure assimilation (biodisponibilité) de ces composants et donc leur action bénéfique sur le follicule, et /ou
- à une extraction sélective de certains composants présents dans la racine d'ortie par l'huile de pépins de courge et l'huile de bourrache émulsifiées par la lécithine, et/ou
- à une synergie d'activité de chacun des composants nécessaires pour observer une amélioration clinique significative, et/ou
- à la capacité des composants contenus dans la formule à exercer une activité anti-inflammatoire et/ou à moduler l'immunité.

## Revendications

1. Composition comprenant en association :
(a) au moins une huile de courge, et
(b) au moins une huile de bourrache.

2. Composition selon la revendication 1, **caractérisée en ce qu'**elle comprend en outre au moins un composé choisi parmi un extrait d'ortie (c) et un extrait de soja (d).

3. Composition selon la revendication 1 ou 2, **caractérisée en ce que** l'huile de courge (a) est une huile extraite de pépins de courge et/ou l'huile de bourrache (b) est une huile extraite de graines de bourrache.

4. Composition selon l'une quelconque des revendications 1 à 3, **caractérisée en ce que** l'extrait d'ortie (c) est un extrait de racines d'ortie, avantageusement sous forme de poudre.

5. Composition selon l'une quelconque des revendications 1 à 4, **caractérisée en ce que** l'extrait de soja (d) est de la lécithine de soja ou un extrait riche en isoflavones, avantageusement sous forme de poudre.

6. Composition selon l'une quelconque des revendications précédentes, **caractérisée en ce que** l'huile de courge (a) est présente à une concentration comprise entre 20 et 40 %, avantageusement entre 25 et 35 % en poids, par rapport au poids total de la composition.

7. Composition selon l'une quelconque des revendications précédentes, **caractérisée en ce que** l'huile de bourrache (b) est présente à une concentration comprise entre 10 et 30 %, avantageusement entre 15 et 30 % en poids, par rapport au poids total de la composition.

8. Composition selon l'une quelconque des revendications précédentes, **caractérisée en ce que** l'extrait d'ortie (c) est présent à une concentration comprise entre 0 et 60 %, avantageusement entre 35 et 55 % en poids, par rapport au poids total de la composition.

9. Composition selon l'une quelconque des revendications précédentes, **caractérisée en ce que** l'extrait de soja (d) est présent à une concentration comprise entre 0 et 10 %, avantageusement entre 0 et 5 % en poids, par rapport au poids total de la composition.

10. Composition selon l'une quelconque des revendications précédentes, **caractérisée en ce que** la composition est formulée pour être administrée par voie orale.

11. Composition selon la revendication 10, **caractérisée en ce que** la composition se présente sous forme de capsule, gélule, comprimé, granule, pâte à mâcher, gomme, huile ou gelée huileuse.

12. Composition selon la revendication 10, **caractérisée en ce que** la composition est une composition ou un complément alimentaire.

13. Composition selon l'une quelconque des revendications 1 à 9, **caractérisée en ce que** la composition est formulée pour être administrée par voie topique ou rectale.

14. Composition selon l'une quelconque des revendications 4 à 13, **caractérisée en ce qu'**elle est susceptible d'être obtenue selon le procédé comprenant la macération de la poudre d'ortie (c) dans un mélange d'huile de courge (a) et d'huile de bourrache (b), éventuellement additionné de lécithine de soja.

15. Composition selon l'une quelconque des revendications précédentes pour son utilisation comme médicament, comme agent dermatologique ou dermo-cosmétique, ou comme nutraceutique.

16. Composition selon la revendication 15 pour son utilisation dans la prévention ou le traitement de l'alopécie, de l'acné, de l'hirsutisme, de la séborrhée ou des odeurs corporelles.

## Claims

1. Composition comprising, in combination:
(a) at least one marrow oil, and
(b) at least one borage oil.

2. Composition according to Claim 1, **characterized in that** it also comprises at least one compound chosen from a nettle extract (c) and a soybean extract (d).

3. Composition according to Claim 1 or 2, **characterized in that** the marrow oil (a) is an oil extracted from marrow pips and/or the borage oil (b) is an oil extracted from borage seeds.

4. Composition according to any one of Claims 1 to 3, **characterized in that** the nettle extract (c) is an extract of nettle roots, advantageously in powder form.

5. Composition according to any one of Claims 1 to 4, **characterized in that** the soybean extract (d) is soybean lecithin or an isoflavone-rich extract, advantageously in powder form.

6. Composition according to any one of the preceding claims, **characterized in that** the marrow oil (a) is present in a concentration of between 20% and 40% and advantageously between 25% and 35% by weight relative to the total weight of the composition.

7. Composition according to any one of the preceding claims, **characterized in that** the borage oil (b) is present in a concentration of between 10% and 30% and advantageously between 15% and 30% by weight relative to the total weight of the composition.

8. Composition according to any one of the preceding claims, **characterized in that** the nettle extract (c) is present in a concentration of between 0 and 60% and advantageously between 35% and 55% by weight relative to the total weight of the composition.

9. Composition according to any one of the preceding claims, **characterized in that** the soybean extract (d) is present in a concentration of between 0 and 10% and advantageously between 0 and 5% by weight relative to the total weight of the composition.

10. Composition according to any one of the preceding claims, **characterized in that** the composition is formulated for oral administration.

11. Composition according to Claim 10, **characterized in that** the composition is in the form of a wafer capsule, a gel capsule, a tablet, a granule, a chewing paste, a gum, an oil or an oily jelly.

12. Composition according to Claim 10, **characterized in that** the composition is a food composition or a food supplement.

13. Composition according to any one of Claims 1 to 9, **characterized in that** the composition is formulated for topical or rectal administration.

14. Composition according to any one of Claims 4 to 13, **characterized in that** it can be obtained according to the process comprising the maceration of the nettle powder (c) in a mixture of marrow oil (a) and borage oil (b), to which soybean lecithin is optionally added.

15. Composition according to any one of the preceding claims, for its use as a medicinal product, as a dermatological or dermocosmetic agent, or as a nutraceutical agent.

16. Composition according to Claim 15, for its use in the prevention or treatment of alopecia, acne, hirsutism, seborrhoea or body odour.

## Patentansprüche

1. Zusammensetzung, umfassend in Kombination:
(a) wenigstens ein Kürbisöl und
(b) wenigstens ein Borretschöl.

2. Zusammensetzung nach Anspruch 1, **dadurch gekennzeichnet, dass** sie außerdem wenigstens eine Verbindung, welche unter einem Brennnesselextrakt (c) und einem Sojaextrakt (d) ausgewählt wird, umfasst.

3. Zusammensetzung nach Anspruch 1 oder 2, **dadurch gekennzeichnet, dass** das Kürbisöl (a) ein Öl, welches aus Kürbiskernen extrahiert wird, ist und/oder das Borretschöl (b) ein Öl, welches aus Borretschsamen extrahiert wird, ist.

4. Zusammensetzung nach einem der Ansprüche 1 bis 3, **dadurch gekennzeichnet, dass** der Brennnesselextrakt (c) ein Extrakt von Brennnesselwurzeln, vorteilhafterweise in Form von Pulver, ist.

5. Zusammensetzung nach einem der Ansprüche 1 bis 4, **dadurch gekennzeichnet, dass** der Sojaextrakt (d) Sojalecithin oder ein an Isoflavonen reicher Extrakt, vorteilhafterweise in Form von Pulver, ist.

6. Zusammensetzung nach einem der vorangegangenen Ansprüche, **dadurch gekennzeichnet, dass** das Kürbisöl (a) in einer Konzentration zwischen 20 und 40 Gew.-% eingeschlossen, vorteilhafterweise zwischen 25 und 35 Gew.-%, bezogen auf das Gesamtgewicht der Zusammensetzung vorliegt.

7. Zusammensetzung nach einem der vorangegangenen Ansprüche, **dadurch gekennzeichnet, dass** das Borretschöl (b) in einer Konzentration zwischen 10 und 30 Gew.-% eingeschlossen, vorteilhafterweise zwischen 15 und 30 Gew.-%, bezogen auf das Gesamtgewicht der Zusammensetzung vorliegt.

8. Zusammensetzung nach einem der vorangegangenen Ansprüche, **dadurch gekennzeichnet, dass** der Brennnesselextrakt (c) in einer Konzentration zwischen 0 und 60 Gew.-% eingeschlossen, vorteilhafterweise zwischen 35 und 55 Gew.-%, bezogen auf das Gesamtgewicht der Zusammensetzung vorliegt.

9. Zusammensetzung nach einem der vorangegangenen Ansprüche, **dadurch gekennzeichnet, dass** der Sojaextrakt (d) in einer Konzentration zwischen 0 und 10 Gew.-% eingeschlossen, vorteilhafterweise zwischen 0 und 5 Gew.-%, bezogen auf das Gesamtgewicht der Zusammensetzung vorliegt.

10. Zusammensetzung nach einem der vorangegangenen Ansprüche, **dadurch gekennzeichnet, dass** die Zusammensetzung formuliert ist, um auf oralem Wege verabreicht zu werden.

11. Zusammensetzung nach Anspruch 10, **dadurch gekennzeichnet, dass** die Zusammensetzung in Form einer Kapsel, Hartkapsel, Tablette, eines Granulats, einer zu kauenden Paste, eines Gummis, Öls oder ölartigen Gelees vorliegt.

12. Zusammensetzung nach Anspruch 10, **dadurch gekennzeichnet, dass** die Zusammensetzung eine Nahrungsmittelzusammensetzung oder eine Nahrungsergänzung ist.

13. Zusammensetzung nach einem der Ansprüche 1 bis 9, **dadurch gekennzeichnet, dass** die Zusammensetzung formuliert ist, um auf topischem oder rektalem Wege verabreicht zu werden.

14. Zusammensetzung nach einem der Ansprüche 4 bis 13, **dadurch gekennzeichnet, dass** sie erhalten werden kann gemäß dem Verfahren, welches die Mazeration des Pulvers von Brennnessel (c) in einer Mischung von Kürbisöl (a) und von Borretschöl (b), zu welcher gegebenenfalls Sojalecithin hinzugesetzt ist, umfasst.

15. Zusammensetzung nach einem der vorangegangenen Ansprüche für deren Verwendung als Arzneimittel, als dermatologisches oder dermo-kosmetisches Mittel oder als Nutrazeutikum.

16. Zusammensetzung nach Anspruch 15 für deren Verwendung bei der Verhütung oder Behandlung von Alopezie, Akne, Hirsutismus, Seborrhoe oder Körpergerüchen.
